# EUROPÄISCHE PATENTSCHRIFT

(11) **EP 0 971 884 B1**
(45) Veröffentlichungstag und Bekanntmachung des Hinweises auf die Patenterteilung: **27.11.2002**
(21) Anmeldenummer: 98913677.5
(22) Anmeldetag: 06.03.1998
(51) Int. Cl.: C07C 251/60, A01N 37/50

(54) **PHENYLKETIMINOOXYBENZYLVERBINDUNGEN, VERFAHREN ZU IHRER HERSTELLUNG UND IHRE VERWENDUNG**
PHENYLKETIMINOOXYBENZYL COMPOUNDS, METHOD FOR THE PRODUCTION AND USE THEREOF
COMPOSES DE PHENYLCETIMINOOXYBENZYLE, LEUR PROCEDE DE PRODUCTION ET LEUR UTILISATION

(30) Priorität: 18.03.1997 DE 19711168
(43) Veröffentlichungstag der Anmeldung: 19.01.2000
(73) Patentinhaber: BASF AKTIENGESELLSCHAFT, 67056 Ludwigshafen (DE)
(72) Erfinder: GRAMMENOS, Wassilios, D-67063 Ludwigshafen (DE); SAUTER, Hubert, D-68167 Mannheim (DE); BAYER, Herbert, D-68159 Mannheim (DE); GROTE, Thomas, D-67105 Schifferstadt (DE); GYPSER, Andreas, D-68159 Mannheim (DE); KIRSTGEN, Reinhard, D-67434 Neustadt (DE); MÜLLER, Bernd, D-67227 Frankenthal (DE); PTOCK, Arne, D-67067 Ludwigshafen (DE); RÖHL, Franz, D-67105 Schifferstadt (DE); GÖTZ, Roland, D-91541 Rothenburg (DE); LORENZ, Gisela, D-67434 Neustadt (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(74) Vertreter: Fitzner, Ulrich, Dr.
(86) Internationale Anmeldenummer: EP9801323
(87) Internationale Veröffentlichungsnummer: WO98041498

(56) Entgegenhaltungen:
- EP-A- 0 472 300
- EP-A- 0 585 751
- WO-A-92/13830

## Beschreibung

Die vorliegende Erfindung betrifft Phenylketiminooxybenzylverbindungen der allgemeinen Formel I 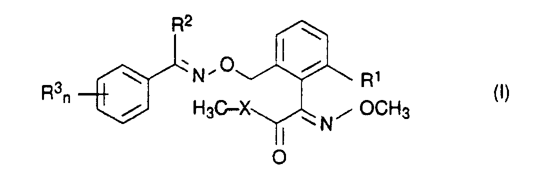 in der die Substituenten und der Index die folgende Bedeutung haben:
- X: O oder NH;
- R¹: Fluor, Chlor, C₁-C₄-Alkyl, C₁-C₄-Halogenalkyl, C₁-C₄-Alkoxy und C₁-C₄-Halogenalkoxy;
- R²: Halogen, C₁-C₄-Alkyl, C₁-C₄-Halogenalkyl und C₁-C₄-Alkoxy;
- R³: Halogen, C₁-C₄-Alkyl, C₁-C₄-Halogenalkyl, C₁-C₄-Alkoxy, C₁-C₄-Halogenalkoxy, C₃-C₆-Cycloalkyl, Phenyl oder Phenoxy, wobei die Phenylreste jeweils ihrerseits eine bis drei der folgenden Gruppen tragen können: Halogen, C₁-C₄-Alkyl, C₁-C₄-Halogenalkyl, C₁-C₄-Alkoxy und C₁-C₄-Halogenalkoxy;
CR^{a}=NOR^{b}, wobei
R^{a} für Wasserstoff oder C₁-C₄-Alkyl steht, und
R^{b} für C₁-C₄-Alkyl, C₃-C₄-Alkenyl oder C₃-C₄-Alkinyl steht;
- n: 1, 2 oder 3, wobei die Reste R³ verschieden sein können, wenn n 2 oder 3 bedeutet,
sowie ihre Salze.

Außerdem betrifft die Erfindung Verfahren zur Herstellung der Verbindungen I sowie sie enthaltende Mittel und ihre Verwendung zur Bekämpfung von tierischen Schädlingen und Schadpilzen.

Aus der Literatur sind Wirkstoffe bekannt, deren allgemeine Formel sich von den vorliegenden Verbindungen I dadurch unterscheidet, daß sie in der Position von R¹ nicht substituiert sind (EP-A 460 575; EP-A 585 751). Außerdem werden in der Literatur Wirkstoffe beschrieben, deren allgemeine Formel die vorliegenden Verbindungen I umfaßt (EP-A 463 488; EP-A 472 300; WO-A 92/13,830).

Der vorliegenden Erfindung lagen demgegenüber Verbindungen als Aufgabe zugrunde, die ein breiteres Wirkungsspektrum und eine verbesserte Wirkung zeigen.

Demgemäß wurden die eingangs definierten Verbindungen I gefunden. Außerdem wurden Verfahren zur Herstellung der Verbindungen I sowie sie enthaltende Mittel und ihre Verwendung zur Bekämpfung von tierischen Schädlingen und Schadpilzen gefunden.

Die vorliegenden Verbindungen I unterscheiden sich von den aus EP-A 463 488, EP-A 472 300 und WO-A 92/13,830 bekannten Verbindungen durch die besondere Kombination der Gruppen R¹ und R³. Insbesondere wurde gefunden, daß Verbindungen des bekannten Strukturtyps eine verbesserte Wirkung zeigen, wenn sie eine der unter R¹ genannten Gruppen in der 6-Position tragen und der Phenylrest zusätzlich durch mindestens einen Rest R³ substituiert ist, der die Lipophilie der Verbindung erhöht.

Die Verbindungen I können im allgemeinen nach den in der eingangs zitierten Literatur beschriebenen Verfahren erhalten werden.

Besonders vorteilhaft erhält man die Verbindungen I dadurch, daß man ein N-Hydroxyketimin IIa in einem inerten Lösungsmittel mit einer Benzylverbindung IIIa umsetzt. 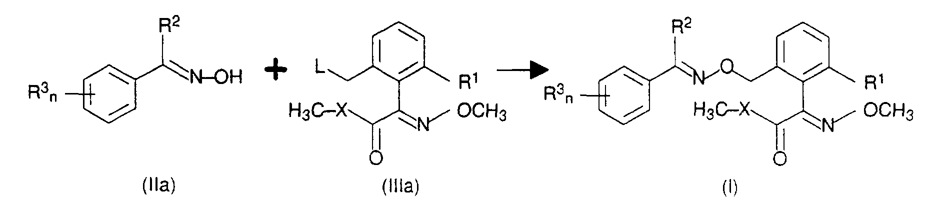 L in der Formel IIIa steht für eine übliche Abgangsgruppe steht, z.B. Halogen (Fluor, Chlor, Brom, Iod, insbesondere Chlor und Brom), C₁-C₄-Alkylsulfonyl (insbesondere Mesylat), C₁-C₄-Halogenalkylsulfonyl (insbesondere Triflat) oder Arylsulfonyl (insbesondere Tosylat).

Die Umsetzung von IIa mit IIIa erfolgt üblicherweise bei Temperaturen von -30°C bis 120°C, vorzugsweise -10°C bis 90°C, in einem inerten organischen Lösungsmittel in Gegenwart einer Base (vgl. Lit. DE-A 40 20 384).

Geeignete Lösungsmittel sind Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Tetrahydrofuran, Acetonitril, Aceton und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetallund Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriummethanolat und Natriumhydrid.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, IIa in einem Überschuß bezogen auf IIIa einzusetzen.

Die für die Herstellung der Verbindungen I benötigten Ausgangsstoffe IIa sind in der Literatur bekannt [Bull. Soc. Chim. Fr. 69 (1899); Synth Commun. 1247 (1986); J. Agric. Food Chem. 923 (1969)] oder können gemäß der zitierten Literatur hergestellt werden.

Die Ausgangsstoffe der Formel IIIa, in denen X für O steht, erhält man vorteilhaft dadurch, daß man ein 1-Brom-2-methylbenzol der Formel IV mit Oxalsäuremethylester-chlorid in den entsprechenden α-Keto-phenylessigsäure-ester der Formel V überführt, V in an sich bekannter Weise mit O-Methyl-hydroxylamin in das entsprechende Oxim VI überführt und VI anschließend zu IIIa halogeniert.

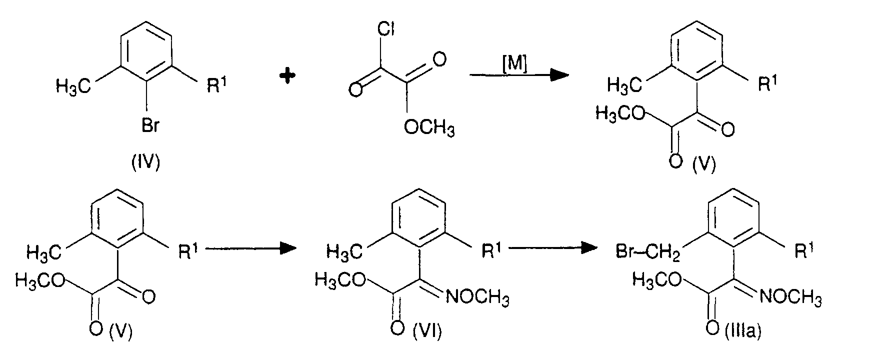

Die Umsetzung von IV mit Oxalsäuremethylester-chlorid erfolgt üblicherweise bei Temperaturen von -78°C bis 80°C, vorzugsweise -10°C bis 60°C, in einem inerten organischen Lösungsmittel in Gegenwart eines Hilfsreagens [M].

Als Hilfsreagens [M] finden Metalle wie Magnesium oder Metallorganyle wie Methyl-Lithium, Butyl-Lithium und Phenyl-Lithium Verwendung. Bevorzugt werden Magnesium und Butyl-Lithium verwendet. Das Hilfsreagens wird im allgemeinen mindestens in stöchiometrischen Mengen eingesetzt. Vorzugsweise verwendet man es in Mengen von 1 mol-Äq. bis 2 mol-Äq., insbesondere 1 mol-Äq bis 1,3 mol-Äq., bezogen auf das Phenyderivat IV.

Geeignete Lösungsmittel sind Ether wie Diethylether, Diisopropylether, tert.-Butylmethylether, Dioxan, Anisol und Tetrahydrofuran, besonders bevorzugt Dimethylether oder Tetrahydrofuran. Es können auch Gemische der genannten Lösungsmittel verwendet werden.

Die Edukte werden im allgemeinen in äquimolaren Mengen miteinander umgesetzt. Es kann für die Ausbeute vorteilhaft sein, Oxalsäuremethylester-chlorid in einem Überschuß bezogen auf IV einzusetzen.

Die Umsetzung des α-Ketoesters V mit O-Methylhydroxylamin erfolgt in an sich bekannter Weise bei Temperaturen von 0°C bis 90°C, vorzugsweise 20°C bis 70°C, in einem inerten organischen Lösungsmittel im allgemeinen und im besonderen gemäß den in EP-A-493 711 beschriebenen Bedingungen.

Die Halogenierung von VI zu IIIa erfolgt in an sich bekannter Weise bei Temperaturen von 0°C bis 120°C, vorzugsweise 20°C bis 110°C, in einem inerten organischen Lösungsmittel im allgemeinen und im besonderen gemäß den in EP-A 385 224 beschriebenen Bedingungen.

Die Verbindungen IIIa, in denen X für NH steht, erhält man vorteilhaft aus den entsprechenden Estern VI durch Umsetzung mit Methylamin oder dessen Salz und anschließende Halogenierung.

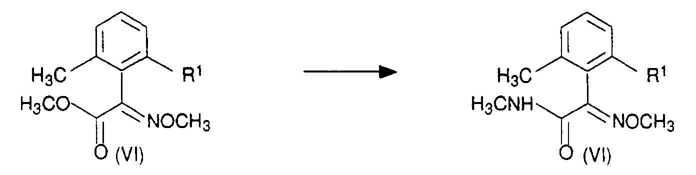

Die Umsetzung des Esters zum Amid erfolgt in an sich bekannter Weise bei Temperaturen von 0°C bis 90°C, vorzugsweise 20°C bis 70°C, in einem inerten organischen Lösungsmittel im allgemeinen und im besonderen gemäß den in EP-A 477 631 und DE-A 40 20 384 beschriebenen Bedingungen.

Die Halogenierung der Amide VI erfolgt im allgemeinen und im besonderen unter den für die Halogenierung der Ester beschriebenen Bedingungen.

Verbindungen IIIa, in denen L für eine andere Abgangsgruppe als Halogen steht, erhält man aus den entsprechenden Halogeniden durch übliche Veretherung mit einem Sulfonat [vgl.: J. Am. Chem. Soc. 4113 (1959); HU-A 182 858].

Nach einem weiteren Verfahren erhält man die Verbindungen I, in denen R² für Alkyl oder Halogenalkyl steht, auch dadurch, daß man ein Phenylketon IIb in einem inerten Lösungsmittel mit einem O-Benzylhydroxylamin IIIb umsetzt.

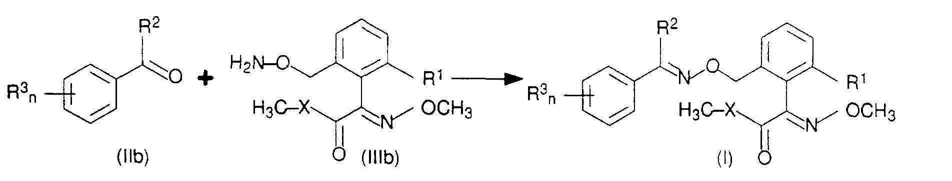

Diese Umsetzung erfolgt in an sich bekannter Weise bei Temperaturen von -50°C bis 90°C, vorzugsweise 0°C bis 70°C, in einem inerten organischen Lösungsmittel in Gegenwart einer Base im allgemeinen und im besonderen gemäß den in DE-A 40 20 384 beschriebenen Bedingungen.

Die Edukte werden im allgemeinen in äquimolaren Mengen miteinander umgesetzt. Es kann für die Ausbeute vorteilhaft sein, IIb in einem Überschuß bezogen auf IIIb einzusetzen.

Die für die Herstellung der Verbindungen I benötigten Ketone IIb sind im Handel erhältlich, aus der Literatur bekannt oder können nach allgemein üblichen Verfahren erhalten werden.

Die 0-Benzyl-hydroxylamine IIIb erhält man vorteilhaft dadurch, daß man eine Verbindung IIIa zunächst mit N-Hydroxyphthalimid verethert und den danach erhaltenen Benzylether VII in an sich bekannter Weise spaltet.

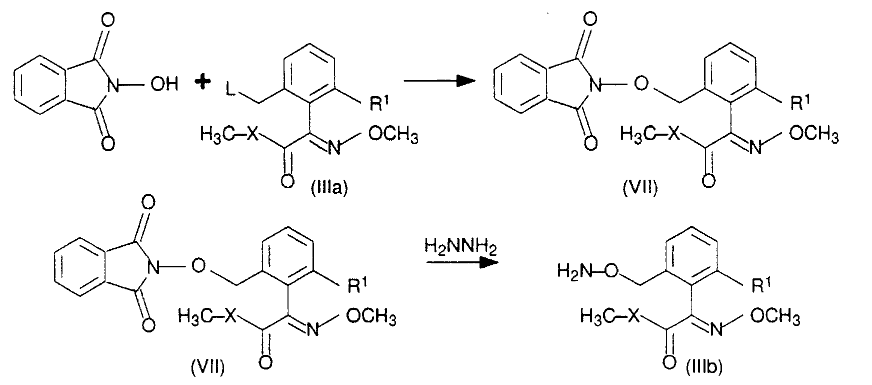

Die Umsetzung der Benzylverbindung mit N-Hydroxy-phthalimid erfolgt in an sich bekannter Weise bei Temperaturen von -30°C bis 100°C, vorzugsweise 0°C bis 70°C, in einem inerten organischen Lösungsmittel in Gegenwart einer Base im allgemeinen und im besonderen gemäß den in DE-A 40 20 388 beschriebenen Bedingungen.

Die Hydrolyse des Benzylethers VII erfolgt in an sich bekannter Weise bei Temperaturen von -30°C bis 90°C, vorzugsweise -10°C bis 70°C, in einem inerten organischen Lösungsmittel im allgemeinen und im besonderen gemäß den in DE-A 40 20 388 beschriebenen Bedingungen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=N-OCH₃-Doppelbindung werden hinsichtlich ihrer Wirksamkeit die E-Isomere der Verbindungen I bevorzugt (Konfiguration bezogen auf die OCH₃ bzw. CH₃-Gruppe im Verhältnis zur COXCH₃-Gruppe). Sofern bei der Synthese Isomerengemische bezüglich dieser Doppelbindung gebildet werden, können die weitgehend reinen E-Isomere dadurch erhalten werden, daß man das E/Z-Isomerengemisch mit HCl in Methanol behandelt [vgl. EP-A 493 711]. Die Isomerisierung der C=N-OCH₃-Doppelbindung kann sowohl auf der Stufe der Verbindungen I als auch auf der Stufe der Vorprodukte der Formeln VI und IIIa erfolgen. Besonders bevorzugt führt man die Isomerisierung auf der Stufe der Verbindungen VI durch.

In Bezug auf die N=CR²-Doppelbindung werden im allgemeinen hinsichtlich ihrer Wirksamkeit die E-Isomere der Verbindungen I (Konfiguration bezogen auf den Phenyl-Rest im Verhältnis zur OCH₂-Gruppe) bevorzugt.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**C**_{**1**}**-C**_{**4**}**-Alkyl:** gesättigte, geradkettige oder verzweigte Kohlenwasserstoffreste mit 1 bis 4 Kohlenstoffatomen, wie Methyl, Ethyl, Propyl, 1-Methylethyl, Butyl, 1-Methylpropyl, 2-Methylpropyl und 1,1-Dimethylethyl;
**C**_{**1**}**-C**_{**4**}**-Halogenalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**C**_{**1**}**-C**_{**4**}**-Alkoxy:** geradkettige oder verzweigte Alkylgruppen mit 1 bis 4 Kohlenstoffatomen (wie vorstehend genannt), welche über ein Sauerstoffatom (-O-) an das Gerüst gebunden sind;
**C**_{**1**}**-C**_{**4**}**-Halogenalkoxy:** geradkettige oder verzweigte Halogenalkylgruppen mit 1 bis 4 Kohlenstoffatomen (wie vorstehend genannt), welche über ein Sauerstoffatom (-O-) an das Gerüst gebunden sind;
**C**_{**3**}**-C**_{**4**}**-Alkenyl:** ungesättigte, geradkettige oder verzweigte Kohlenwasserstoffreste mit 3 oder 4 Kohlenstoffatomen und einer Doppelbindung in einer beliebigen Position, wie 2-Propenyl, 2-Butenyl, 3-Butenyl, 1-Methyl-2-propenyl und 2-Methyl-2-propenyl;
**C**_{**3**}**-C**_{**4**}**-Alkinyl:** geradkettige oder verzweigte Kohlenwasserstoffgruppen mit 3 oder 4 Kohlenstoffatomen und einer Dreifachbindung in einer beliebigen Position, wie 2-Propinyl, 2-Butinyl, 3-Butinyl und 1-Methyl-2-propinyl;
**C**_{**3**}**-C**_{**6**}**-Cycloalkyl:** monocyclische, gesättigte Kohlenwasserstoffgruppen mit 3 bis 6 Kohlenstoffringgliedern, wie Cyclopropyl, Cyclobutyl, Cyclopentyl und Cyclohexyl.

Im Hinblick auf ihre biologische Wirkung sind Verbindungen I bevorzugt, in denen X Sauerstoff bedeutet.

Desweiteren werden Verbindungen I bevorzugt, in denen X für NH steht.

Im Hinblick auf die Gruppierung R¹ werden Verbindungen I bevorzugt, die in dieser Position eine der folgenden Gruppen tragen: Fluor, Chlor, C₁-C₂-Alkyl, C₁-C₂-Halogenalkyl, C₁-C₂-Alkoxy und C₁-C₂-Halogenalkoxy.

Insbesondere werden Verbindungen I bevorzugt, in denen R¹ für Fluor oder Chlor steht.

Außerdem werden Verbindungen I bevorzugt, in denen R¹ für C₁-C₂-Alkyl (insbesondere Methyl), C₁-C₂-Halogenalkyl (insbesondere Trifluormethyl), C₁-C₂-Alkoxy (insbesondere Methoxy) und C₁-C₂-Halogenalkoxy (insbesondere Trifluormethoxy und Difluormethoxy) steht.

Im Hinblick auf die Gruppierung R² werden Verbindungen I bevorzugt, die in dieser Position eine der folgenden Gruppen tragen: Fluor, Chlor, Brom, C₁-C₂-Alkyl, C₁-C₂-Halogenalkyl und C₁-C₄-Alkoxy.

Insbesondere werden Verbindungen I bevorzugt, in denen R² für Fluor, Chlor oder Brom (insbesondere Chlor) steht.

Daneben werden Verbindungen I bevorzugt, in denen R² für C₁-C₂-Alkyl (insbesondere Methyl), C₁-C₂-Halogenalkyl (insbesondere Trifluormethyl) und C₁-C₄-Alkoxy (besonders Methoxy, Ethoxy und iso-Propyloxy, insbesondere Methoxy) steht.

Außerdem werden Verbindungen I bevorzugt, welche am Phenylring eine Gruppe R³ in der 3- oder 4-Position (insbesondere in 3-Position) tragen.

Daneben werden Verbindungen I bevorzugt, welche am Phenylring zwei Gruppen R³ in den Positionen 3 und 4 oder 3 und 5 tragen.

Desweiteren werden Verbindungen I bevorzugt, welche am Phenylring drei Gruppen R³ in den Positionen 2, 4 und 5 tragen.

Im Hinblick auf die Gruppierung R³ werden Verbindungen I bevorzugt, die in dieser Position eine bis drei der folgenden Gruppen tragen:
- Halogen (besonders Fluor oder Chlor, insbesondere Chlor), C₁-C₂-Alkyl (insbesondere Methyl), C₁-C₂-Halogenalkyl (besonders Difluormethyl und Trifluormethyl, insbesondere Trifluormethyl), C₁-C₃-Alkoxy (besonders Methoxy und iso-Propyloxy, insbesondere Methoxy), C₁-C₂-Halogenalkoxy (besonders Difluormethoxy und Trifluormethoxy, insbesondere Trifluormethoxy) und C₃-C₆-Cycloalkyl (insbesondere Cyclopropyl),
und/oder eine der folgenden Gruppen steht:
- Phenyl oder Phenoxy, wobei die Phenylreste jeweils ihrerseits eine bis drei der folgenden Gruppen tragen können: Halogen (besonders Fluor oder Chlor, insbesondere Chlor), C₁-C₂-Alkyl (insbesondere Methyl), C₁-C₂-Halogenalkyl (besonders Difluormethyl und Trifluormethyl, insbesondere Trifluormethyl), C₁-C₃-Alkoxy (besonders Methoxy und iso-Propyloxy, insbesondere Methoxy) und C₁-C₂-Halogenalkoxy (besonders Difluormethoxy und Trifluormethoxy, insbesondere Trifluormethoxy) ;
- CR^{a}=NOR^{b}, wobei R^{a} für Wasserstoff oder C₁-C₄-Alkyl (besonders C₁-C₃-Alkyl, insbesondere Methyl) steht, und R^{b} für C₁-C₄-Alkyl (besonders C₁-C₃-Alkyl, insbesondere Methyl, Ethyl und iso-Propyl), C₃-C₄-Alkenyl (besonders Allyl und 2-Butenyl) oder C₃-C₄-Alkinyl (besonders Propargyl und 2-Butinyl).

Insbesondere werden Verbindungen I im Hinblick auf die biologische Wirkung bevorzugt, in denen die Substituenten die folgende Bedeutung haben:
- X: O oder NH;
- R¹: Fluor, Chlor, Methyl, Trifluormethyl, Methoxy und Trifluormethoxy;
- R²: Fluor, Chlor, Methyl, Trifluormethyl und Methoxy;
- R³: Fluor, Chlor, Methyl, Trifluormethyl, Methoxy, Trifluormethoxy, Cyclopropyl, Phenyl oder Phenoxy, wobei die Phenylreste jeweils ihrerseits eine bis drei der folgenden Gruppen tragen können: Fluor, Chlor, Methyl, Trifluormethyl, Methoxy und Trifluormethoxy;
CH=NOCH₃, CH=NOCH₂CH₃, CH=NOCH₂CH₂CH₃, CH=NOCH(CH₃)₂, CH=NOCH₂CH=CH₂, CH=NOCH₂CH=CHCH₃, CH=NOCH₂CH≡CH, CH=NOCH₂CH≡CCH₃, C(CH₃)=NOCH₃, C(CH₃)=NOCH₂CH₃, C(CH₃)=NOCH₂CH₂CH₃, C(CH₃)=NOCH(CH₃)₂, C(CH₃)=NOCH₂CH=CH₂, C(CH₃)=NOCH₂CH=CHCH₃, C(CH₃)=NOCH₂CH≡CH und C(CH₃)=NOCH₂CH≡CCH₃;
- n: 1, 2 oder 3, wobei die Reste R³ verschieden sein können, wenn n 2 oder 3 bedeutet.

Im Hinblick auf ihre biologische Aktivität werden insbesondere die in den folgenden Tabellen zusammengestellten Verbindungen I bevorzugt. Die in den Tabellen für einen Substituenten genannten Gruppen stellen außerdem, für sich betrachtet, unabhängig von der Kombination in der sie genannt sind, eine besonders bevorzugte Ausgestaltung des betreffenden Substituenten dar.

### Tabelle 1

Verbindungen der allgemeinen Formel I.A, in denen R¹ für Methyl steht, R² Chlor bedeutet und die Kombination der Reste R³ₙ für eine Verbindung jeweils einer Zeile der Tabelle A entspricht

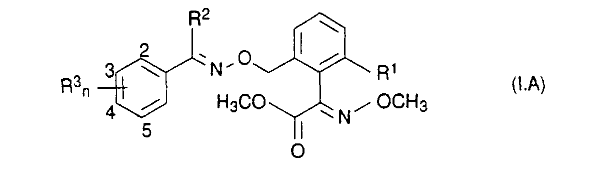

### Tabelle 2

Verbindungen der allgemeinen Formel I.B, in denen R¹ für Methyl steht, R² Chlor bedeutet und die Kombination der Reste R³ₙ für eine Verbindung jeweils einer Zeile der Tabelle A entspricht

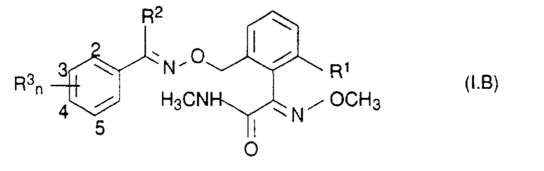

### Tabelle 3

Verbindungen der allgemeinen Formel I.A, in denen R¹ für Fluor steht, R² Methyl bedeutet und die Kombination der Reste R³ₙ für eine Verbindung jeweils einer Zeile der Tabelle A entspricht

### Tabelle 4

Verbindungen der allgemeinen Formel I.B, in denen R¹ für Fluor steht, R² Methyl bedeutet und die Kombination der Reste R³ₙ für eine Verbindung jeweils einer Zeile der Tabelle A entspricht

### Tabelle 5

Verbindungen der allgemeinen Formel I.A, in denen R¹ für Chlor steht, R² Methyl bedeutet und die Kombination der Reste R³ₙ für eine Verbindung jeweils einer Zeile der Tabelle A entspricht

### Tabelle 6

Verbindungen der allgemeinen Formel I.B, in denen R¹ für Chlor steht, R² Methyl bedeutet und die Kombination der Reste R³ₙ für eine Verbindung jeweils einer Zeile der Tabelle A entspricht

### Tabelle 7

Verbindungen der allgemeinen Formel I.A, in denen R¹ für Methyl steht, R² Methyl bedeutet und die Kombination der Reste R³ₙ für eine Verbindung jeweils einer Zeile der Tabelle A entspricht

### Tabelle 8

Verbindungen der allgemeinen Formel I.B, in denen R¹ für Methyl steht, R² Methyl bedeutet und die Kombination der Reste R³ₙ für eine Verbindung jeweils einer Zeile der Tabelle A entspricht

### Tabelle 9

Verbindungen der allgemeinen Formel I.A, in denen R¹ für Trifluormethyl steht, R² Methyl bedeutet und die Kombination der Reste R³ₙ für eine Verbindung jeweils einer Zeile der Tabelle A entspricht

### Tabelle 10

Verbindungen der allgemeinen Formel I.B, in denen R¹ für Trifluormethyl steht, R² Methyl bedeutet und die Kombination der Reste R³ₙ für eine Verbindung jeweils einer Zeile der Tabelle A entspricht

### Tabelle 11

Verbindungen der allgemeinen Formel I.A, in denen R¹ für Methoxy steht, R² Methyl bedeutet und die Kombination der Reste R³ₙ für eine Verbindung jeweils einer Zeile der Tabelle A entspricht

### Tabelle 12

Verbindungen der allgemeinen Formel I.B, in denen R¹ für Methoxy steht, R² Methyl bedeutet und die Kombination der Reste R³ₙ für eine Verbindung jeweils einer Zeile der Tabelle A entspricht

### Tabelle 13

Verbindungen der allgemeinen Formel I.A, in denen R¹ für Trifluormethoxy steht, R² Methyl bedeutet und die Kombination der Reste R³ₙ für eine Verbindung jeweils einer Zeile der Tabelle A entspricht

### Tabelle 14

Verbindungen der allgemeinen Formel I.B, in denen R¹ für Trifluormethoxy steht, R² Methyl bedeutet und die Kombination der Reste R³ₙ für eine Verbindung jeweils einer Zeile der Tabelle A entspricht

### Tabelle 15

Verbindungen der allgemeinen Formel I.A, in denen R¹ für Fluor steht, R² Ethyl bedeutet und die Kombination der Reste R³ₙ für eine Verbindung jeweils einer Zeile der Tabelle A entspricht

### Tabelle 16

Verbindungen der allgemeinen Formel I.B, in denen R¹ für Fluor steht, R² Ethyl bedeutet und die Kombination der Reste R³ₙ für eine Verbindung jeweils einer Zeile der Tabelle A entspricht

### Tabelle 17

Verbindungen der allgemeinen Formel I.A, in denen R¹ für Chlor steht, R² Ethyl bedeutet und die Kombination der Reste R³ₙ für eine Verbindung jeweils einer Zeile der Tabelle A entspricht

### Tabelle 18

Verbindungen der allgemeinen Formel I.B, in denen R¹ für Chlor steht, R² Ethyl bedeutet und die Kombination der Reste R³ₙ für eine Verbindung jeweils einer Zeile der Tabelle A entspricht

### Tabelle 19

Verbindungen der allgemeinen Formel I.A, in denen R¹ für Methyl steht, R² Ethyl bedeutet und die Kombination der Reste R³ₙ für eine Verbindung jeweils einer Zeile der Tabelle A entspricht

### Tabelle 20

Verbindungen der allgemeinen Formel I.B, in denen R¹ für Methyl steht, R² Ethyl bedeutet und die Kombination der Reste R³ₙ für eine Verbindung jeweils einer Zeile der Tabelle A entspricht

### Tabelle 21

Verbindungen der allgemeinen Formel I.A, in denen R¹ für Trifluormethyl steht, R² Ethyl bedeutet und die Kombination der Reste R³ₙ für eine Verbindung jeweils einer Zeile der Tabelle A entspricht

### Tabelle 22

Verbindungen der allgemeinen Formel I.B, in denen R¹ für Trifluormethyl steht, R² Ethyl bedeutet und die Kombination der Reste R³ₙ für eine Verbindung jeweils einer Zeile der Tabelle A entspricht

### Tabelle 23

Verbindungen der allgemeinen Formel I.A, in denen R¹ für Methoxy steht, R² Ethyl bedeutet und die Kombination der Reste R³ₙ für eine Verbindung jeweils einer Zeile der Tabelle A entspricht

### Tabelle 24

Verbindungen der allgemeinen Formel I.B, in denen R¹ für Methoxy steht, R² Ethyl bedeutet und die Kombination der Reste R³ₙ für eine Verbindung jeweils einer Zeile der Tabelle A entspricht

### Tabelle 25

Verbindungen der allgemeinen Formel I.A, in denen R¹ für Trifluormethoxy steht, R² Ethyl bedeutet und die Kombination der Reste R³ₙ für eine Verbindung jeweils einer Zeile der Tabelle A entspricht

### Tabelle 26

Verbindungen der allgemeinen Formel I.B, in denen R¹ für Trifluormethoxy steht, R² Ethyl bedeutet und die Kombination der Reste R³ₙ für eine Verbindung jeweils einer Zeile der Tabelle A entspricht

### Tabelle 27

Verbindungen der allgemeinen Formel I.A, in denen R¹ für Methyl steht, R² Methoxy bedeutet und die Kombination der Reste R³ₙ für eine Verbindung jeweils einer Zeile der Tabelle A entspricht

### Tabelle 28

Verbindungen der allgemeinen Formel I.B, in denen R¹ für Methyl steht, R² Methoxy bedeutet und die Kombination der Reste R³ₙ für eine Verbindung jeweils einer Zeile der Tabelle A entspricht

### Tabelle 29

Verbindungen der allgemeinen Formel I.A, in denen R¹ für Trifluormethyl steht, R² Methoxy bedeutet und die Kombination der Reste R³ₙ für eine Verbindung jeweils einer Zeile der Tabelle A entspricht

### Tabelle 30

Verbindungen der allgemeinen Formel I.B, in denen R¹ für Trifluormethyl steht, R² Methoxy bedeutet und die Kombination der Reste R³ₙ für eine Verbindung jeweils einer Zeile der Tabelle A entspricht

### Tabelle 31

Verbindungen der allgemeinen Formel I.A, in denen R¹ für Fluor steht, R² Trifluormethyl bedeutet und die Kombination der Reste R³ₙ für eine Verbindung jeweils einer Zeile der Tabelle A entspricht

### Tabelle 32

Verbindungen der allgemeinen Formel I.B, in denen R¹ für Fluor steht, R² Trifluormethyl bedeutet und die Kombination der Reste R³ₙ für eine Verbindung jeweils einer Zeile der Tabelle A entspricht

### Tabelle 33

Verbindungen der allgemeinen Formel I.A, in denen R¹ für Chlor steht, R² Trifluormethyl bedeutet und die Kombination der Reste R³ₙ für eine Verbindung jeweils einer Zeile der Tabelle A entspricht

### Tabelle 34

Verbindungen der allgemeinen Formel I.B, in denen R¹ für Chlor steht, R² Trifluormethyl bedeutet und die Kombination der Reste R³ₙ für eine Verbindung jeweils einer Zeile der Tabelle A entspricht

### Tabelle 35

Verbindungen der allgemeinen Formel I.A, in denen R¹ für Methyl steht, R² Trifluormethyl bedeutet und die Kombination der Reste R³ₙ für eine Verbindung jeweils einer Zeile der Tabelle A entspricht

### Tabelle 36

Verbindungen der allgemeinen Formel I.B, in denen R¹ für Methyl steht, R² Trifluormethyl bedeutet und die Kombination der Reste R³ₙ für eine Verbindung jeweils einer Zeile der Tabelle A entspricht

**Tabelle A:**

| **Nr.** | **Pos. 2** | **Pos. 3** | **Pos. 4** | **Pos. 5** |
|---|---|---|---|---|
| A.1 | H | F | H | H |
| A.2 | H | Cl | H | H |
| A.3 | H | CH₃ | H | H |
| A.4 | H | CHF₂ | H | H |
| A.5 | H | CF₃ | H | H |
| A.6 | H | OCH₃ | H | H |
| A.7 | H | OCF₃ | H | H |
| A.8 | H | cyclopropyl | H | H |
| A.9 | H | C₆H₅ | H | H |
| A.10 | H | 2-F-C₆H₄ | H | H |
| A.11 | H | 4-F-C₆H₄ | H | H |
| A.12 | H | 2-Cl-C₆H₄ | H | H |
| A.13 | H | 4-Cl-C₆H₄ | H | H |
| A.14 | H | 2-CH₃-C₆H₄ | H | H |
| A.15 | H | 3-CH₃-C₆H₄ | H | H |
| A.16 | H | O-C₆H₅ | H | H |
| A.17 | H | CH=NOCH₃ | H | H |
| A.18 | H | CH=NOCH(CH₃) | H | H |
| A.19 | H | CH=NOCH₂CH=CHCH₃ | H | H |
| A.20 | H | C(CH₃)=NOCH₃ | H | H |
| A.21 | H | C(CH₃)=NOCH₂CH₃ | H | H |
| A.22 | H | C(CH₃)=NOCH₂CH=CHCH₃ | H | H |
| A.23 | H | C(CH₃)=NOCH₂CH≡CCH₃ | H | H |
| A.24 | H | H | F | H |
| A.25 | H | H | Cl | H |
| A.26 | H | H | CH₃ | H |
| A.27 | H | H | CHF₂ | H |
| A.28 | H | H | CF₃ | H |
| A.29 | H | H | OCH₃ | H |
| A.30 | H | H | OCF₃ | H |
| A.31 | H | H | cyclopropyl | H |
| A.32 | H | H | C₆H₅ | H |
| A.33 | H | H | 2-F-C₆H₄ | H |
| A.34 | H | H | 4-F-C₆H₄ | H |
| A.35 | H | H | 2-Cl-C₆H₄ | H |
| A.36 | H | H | 4-Cl-C₆H₄ | H |
| A.37 | H | H | 2-CH₃-C₆H₄ | H |
| A.38 | H | H | 3-CH₃-C₆H₄ | H |
| A.39 | H | H | O-C₆H₅ | H |
| A.40 | H | H | CH=NOCH₃ | H |
| A.41 | H | H | CH=NOCH(CH₃) | H |
| A.42 | H | H | CH=NOCH₂CH=CHCH₃ | H |
| A.43 | H | H | C(CH₃)=NOCH₃ | H |
| A.44 | H | H | C(CH₃)=NOCH₂CH₃ | H |
| A.45 | H | H | C(CH₃)=NOCH₂CH=CHCH₃ | H |
| A.46 | H | H | C(CH₃)=NOCH₂CH≡CCH₃ | H |
| A.47 | H | F | F | H |
| A.48 | H | Cl | F | H |
| A.49 | H | CH₃ | F | H |
| A.50 | H | CHF₂ | F | H |
| A.51 | H | CF₃ | F | H |
| A.52 | H | OCH₃ | F | H |
| A.53 | H | OCHF₂ | F | H |
| A.54 | H | OCF₃ | F | H |
| A.55 | H | cyclopropyl | F | H |
| A.56 | H | C₆H₅ | F | H |
| A.57 | H | O-C₆H₅ | F | H |
| A.58 | H | CH=NOCH₃ | F | H |
| A.59 | H | CH=NOCH(CH₃) | F | H |
| A.60 | H | CH=NOCH₂CH=CHCH₃ | F | H |
| A.61 | H | C(CH₃)=NOCH₃ | F | H |
| A.62 | H | C(CH₃)=NOCH₂CH₃ | F | H |
| A.63 | H | C(CH₃)=NOCH₂CH=CHCH₃ | F | H |
| A.64 | H | C(CH₃)=NOCH₂CH≡CCH₃ | F | H |
| A.65 | H | F | Cl | H |
| A.66 | H | F | CH₃ | H |
| A.67 | H | F | CHF₂ | H |
| A.68 | H | F | CF₃ | H |
| A.69 | H | F | OCH₃ | H |
| A.70 | H | F | OCF₃ | H |
| A.71 | H | F | cyclopropyl | H |
| A.72 | H | F | C₆H₅ | H |
| A.73 | H | F | O-C₆H₅ | H |
| A.74 | H | F | CH=NOCH₃ | H |
| A.75 | H | F | CH=NOCH(CH₃) | H |
| A.76 | H | F | CH=NOCH₂CH=CHCH₃ | H |
| A.77 | H | F | C(CH₃)=NOCH₃ | H |
| A.78 | H | F | C(CH₃)=NOCH₂CH₃ | H |
| A.79 | H | F | C(CH₃)=NOCH₂CH=CHCH₃ | H |
| A.80 | H | F | C(CH₃)=NOCH₂CH≡CCH₃ | H |
| A.81 | H | F | Cl | H |
| A.82 | H | Cl | Cl | H |
| A.83 | H | CH₃ | Cl | H |
| A.84 | H | CHF₂ | Cl | H |
| A.85 | H | CF₃ | Cl | H |
| A.86 | H | OCH₃ | Cl | H |
| A.87 | H | OCHF₂ | Cl | H |
| A.88 | H | OCF₃ | Cl | H |
| A.89 | H | cyclopropyl | Cl | H |
| A.90 | H | C₆H₅ | Cl | H |
| A.91 | H | 2-F-C₆H₄ | Cl | H |
| A.92 | H | 4-F-C₆H₄ | Cl | H |
| A.93 | H | 2-Cl-C₆H₄ | Cl | H |
| A.94 | H | 4-Cl-C₆H₄ | Cl | H |
| A.95 | H | 2-CH₃-C₆H₄ | Cl | H |
| A.96 | H | 3-CH₃-C₆H₄ | Cl | H |
| A.97 | H | O-C₆H₅ | Cl | H |
| A.98 | H | O-[2-F-C₆H₄] | Cl | H |
| A.99 | H | O-[4-F-C₆H₄] | Cl | H |
| A.100 | H | O-[2-Cl-C₆H₄] | Cl | H |
| A.101 | H | O-[4-Cl-C₆H₄] | Cl | H |
| A.102 | H | O-[2-CH₃-C₆H₄] | Cl | H |
| A.103 | H | O-[3-CH₃-C₆H₄] | Cl | H |
| A.104 | H | CH=NOCH₃ | Cl | H |
| A.105 | H | CH=NOCH(CH₃) | Cl | H |
| A.106 | H | CH=NOCH₂CH=CHCH₃ | Cl | H |
| A.107 | H | C(CH₃)=NOCH₃ | Cl | H |
| A.108 | H | C(CH₃)=NOCH₂CH₃ | Cl | H |
| A.109 | H | C(CH₃)=NOCH₂CH=CHCH₃ | Cl | H |
| A.110 | H | C(CH₃)=NOCH₂CH≡CCH₃ | Cl | H |
| A.111 | H | Cl | F | H |
| A.112 | H | Cl | CH₃ | H |
| A.113 | H | Cl | CHF₂ | H |
| A.114 | H | Cl | CF₃ | H |
| A.115 | H | Cl | OCH₃ | H |
| A.116 | H | Cl | OCHF₂ | H |
| A.117 | H | Cl | OCF₃ | H |
| A.118 | H | Cl | cyclopropyl | H |
| A.119 | H | Cl | C₆H₅ | H |
| A.120 | H | Cl | 2-F-C₆H₄ | H |
| A.121 | H | Cl | 4-F-C₆H₄ | H |
| A.122 | H | Cl | 2-Cl-C₆H₄ | H |
| A.123 | H | Cl | 4-Cl-C₆H₄ | H |
| A.124 | H | Cl | 2-CH₃-C₆H₄ | H |
| A.125 | H | Cl | 3-CH₃-C₆H₄ | H |
| A.126 | H | Cl | O-C₆H₅ | H |
| A.127 | H | Cl | CH=NOCH₃ | H |
| A.128 | H | Cl | CH=NOCH(CH₃) | H |
| A.129 | H | Cl | CH=NOCH₂CH=CHCH₃ | H |
| A.130 | H | Cl | C(CH₃)=NOCH₃ | H |
| A.131 | H | Cl | C(CH₃)=NOCH₂CH₃ | H |
| A.132 | H | Cl | C(CH₃)=NOCH₂CH=CHCH₃ | H |
| A.133 | H | Cl | C(CH₃)=NOCH₂CH≡CCH₃ | H |
| A.134 | H | F | CH₃ | H |
| A.135 | H | Cl | CH₃ | H |
| A.136 | H | CH₃ | CH₃ | H |
| A.137 | H | CF₃ | CH₃ | H |
| A.138 | H | OCH₃ | CH₃ | H |
| A.139 | H | OCF₃ | CH₃ | H |
| A.140 | H | cyclopropyl | CH₃ | H |
| A.141 | H | C₆H₅ | CH₃ | H |
| A.142 | H | 2-F-C₆H₄ | CH₃ | H |
| A.143 | H | 2-CH₃-C₆H₄ | CH₃ | H |
| A.144 | H | 3-CF₃-C₆H₄ | CH₃ | H |
| A.145 | H | 4-OCH₃-C₆H₄ | CH₃ | H |
| A.146 | H | 3-OCF₃-C₆H₄ | CH₃ | H |
| A.147 | H | O-C₆H₅ | CH₃ | H |
| A.148 | H | CH=NOCH₃ | CH₃ | H |
| A.149 | H | CH=NOCH(CH₃) | CH₃ | H |
| A.150 | H | CH=NOCH₂CH=CHCH₃ | CH₃ | H |
| A.151 | H | C(CH₃)=NOCH₃ | CH₃ | H |
| A.152 | H | C(CH₃)=NOCH₂CH=CHCH₃ | CH₃ | H |
| A.153 | H | C(CH₃)=NOCH₂CH≡CCH₃ | CH₃ | H |
| A.154 | H | CH₃ | F | H |
| A.155 | H | CH₃ | Cl | H |
| A.156 | H | CH₃ | CF₃ | H |
| A.157 | H | CH₃ | OCH₃ | H |
| A.158 | H | CH₃ | OCF₃ | H |
| A.159 | H | CH₃ | cyclopropyl | H |
| A.160 | H | CH₃ | C₆H₅ | H |
| A.161 | H | CH₃ | O-C₆H₅ | H |
| A.162 | H | CH₃ | CH=NOCH₃ | H |
| A.163 | H | CH₃ | CH=NOCH(CH₃) | H |
| A.164 | H | CH₃ | CH=NOCH₂CH=CHCH₃ | H |
| A.165 | H | CH₃ | C(CH₃)=NOCH₃ | H |
| A.166 | H | CH₃ | C(CH₃)=NOCH₂CH=CHCH₃ | H |
| A.167 | H | CH₃ | C(CH₃)=NOCH₂CH≡CCH₃ | H |
| A.168 | H | F | CF₃ | H |
| A.169 | H | Cl | CF₃ | H |
| A.170 | H | CH₃ | CF₃ | H |
| A.171 | H | CF₃ | CF₃ | H |
| A.172 | H | OCH₃ | CF₃ | H |
| A.173 | H | OCF₃ | CF₃ | H |
| A.174 | H | cyclopropyl | CF₃ | H |
| A.175 | H | C₆H₅ | CF₃ | H |
| A.176 | H | O-C₆H₅ | CF₃ | H |
| A.177 | H | CH=NOCH₃ | CF₃ | H |
| A.178 | H | CH=NOCH(CH₃) | CF₃ | H |
| A.179 | H | CH=NOCH₂CH=CHCH₃ | CF₃ | H |
| A.180 | H | C(CH₃)=NOCH₃ | CF₃ | H |
| A.181 | H | C(CH₃)=NOCH₂CH=CHCH₃ | CF₃ | H |
| A.182 | H | C(CH₃)=NOCH₂CH≡CCH₃ | CF₃ | H |
| A.183 | H | CF₃ | F | H |
| A.184 | H | CF₃ | Cl | H |
| A.185 | H | CF₃ | CH₃ | H |
| A.186 | H | CF₃ | OCH₃ | H |
| A.187 | H | CF₃ | OCF₃ | H |
| A.188 | H | CF₃ | cyclopropyl | H |
| A.189 | H | CF₃ | C₆H₅ | H |
| A.190 | H | CF₃ | O-C₆H₅ | H |
| A.191 | H | CF₃ | CH=NOCH₃ | H |
| A.192 | H | CF₃ | CH=NOCH(CH₃) | H |
| A.193 | H | CF₃ | CH=NOCH₂CH=CHCH₃ | H |
| A.194 | H | CF₃ | C(CH₃)=NOCH₃ | H |
| A.195 | H | CF₃ | C(CH₃)=NOCH₂CH=CHCH₃ | H |
| A.196 | H | CF₃ | C(CH₃)=NOCH₂CH≡CCH₃ | H |
| A.197 | H | F | OCH₃ | H |
| A.198 | H | Cl | OCH₃ | H |
| A.199 | H | CH₃ | OCH₃ | H |
| A.200 | H | CF₃ | OCH₃ | H |
| A.201 | H | OCH₃ | OCH₃ | H |
| A.202 | H | OCF₃ | OCH₃ | H |
| A.203 | H | cyclopropyl | OCH₃ | H |
| A.204 | H | C₆H₅ | OCH₃ | H |
| A.205 | H | O-C₆H₅ | OCH₃ | H |
| A.206 | H | CH=NOCH₃ | OCH₃ | H |
| A.207 | H | CH=NOCH(CH₃) | OCH₃ | H |
| A.208 | H | CH=NOCH₂CH=CHCH₃ | OCH₃ | H |
| A.209 | H | C(CH₃)=NOCH₃ | OCH₃ | H |
| A.210 | H | C(CH₃)=NOCH₂CH=CHCH₃ | OCH₃ | H |
| A.211 | H | C(CH₃)=NOCH₂CH≡CCH₃ | OCH₃ | H |
| A.212 | H | OCH₃ | F | H |
| A.213 | H | OCH₃ | Cl | H |
| A.214 | H | OCH₃ | CH₃ | H |
| A.215 | H | OCH₃ | CF₃ | H |
| A.216 | H | OCH₃ | OCF₃ | H |
| A.217 | H | OCH₃ | cyclopropyl | H |
| A.218 | H | OCH₃ | C₆H₅ | H |
| A.219 | H | OCH₃ | O-C₆H₅ | H |
| A.220 | H | OCH₃ | CH=NOCH₃ | H |
| A.221 | H | OCH₃ | CH=NOCH(CH₃) | H |
| A.222 | H | OCH₃ | CH=NOCH₂CH=CHCH₃ | H |
| A.223 | H | OCH₃ | C(CH₃)=NOCH₃ | H |
| A.224 | H | OCH₃ | C(CH₃)=NOCH₂CH=CHCH₃ | H |
| A.225 | H | OCH₃ | C(CH₃)=NOCH₂CH≡CCH₃ | H |
| A.226 | H | C(CH₃)=NOCH₃ | OCF₃ | H |
| A.227 | H | C(CH₃)=NOCH₂CH=CHCH₃ | OCF₃ | H |
| A.228 | H | C(CH₃)=NOCH₂CH≡CCH₃ | OCF₃ | H |
| A.229 | H | OCF₃ | F | H |
| A.230 | H | OCF₃ | Cl | H |
| A.231 | H | OCF₃ | CH₃ | H |
| A.232 | H | OCF₃ | CF₃ | H |
| A.233 | H | OCF₃ | OCH₃ | H |
| | | | | |
| A.234 | H | F | H | F |
| A.235 | H | Cl | H | F |
| A.236 | H | CH₃ | H | F |
| A.237 | H | CHF₂ | H | F |
| A.238 | H | CF₃ | H | F |
| A.239 | H | OCH₃ | H | F |
| A.240 | H | OCF₃ | H | F |
| A.241 | H | cyclopropyl | H | F |
| A.242 | H | C₆H₅ | H | F |
| A.243 | H | O-C₆H₅ | H | F |
| A.244 | H | CH=NOCH₃ | H | F |
| A.245 | H | CH=NOCH(CH₃) | H | F |
| A.246 | H | CH=NOCH₂CH=CHCH₃ | H | F |
| A.247 | H | C(CH₃)=NOCH₃ | H | F |
| A.248 | H | C(CH₃)=NOCH₂CH₃ | H | F |
| A.249 | H | C(CH₃)=NOCH₂CH=CHCH₃ | H | F |
| A.250 | H | C(CH₃)=NOCH₂CH≡CCH₃ | H | F |
| A.251 | H | F | H | O-C₆H₅ |
| A.252 | H | Cl | H | Cl |
| A.253 | H | CH₃ | H | Cl |
| A.254 | H | CHF₂ | H | Cl |
| A.255 | H | CF₃ | H | Cl |
| A.256 | H | OCH₃ | H | Cl |
| A.257 | H | OCF₃ | H | Cl |
| A.258 | H | cyclopropyl | H | Cl |
| A.259 | H | C₆H₅ | H | Cl |
| A.260 | H | O-C₆H₅ | H | Cl |
| A.261 | H | CH=NOCH₃ | H | Cl |
| A.262 | H | CH=NOCH(CH₃) | H | Cl |
| A.263 | H | CH=NOCH₂CH=CHCH₃ | H | Cl |
| A.264 | H | C(CH₃)=NOCH₃ | H | Cl |
| A.265 | H | C(CH₃)=NOCH₂CH₃ | H | Cl |
| A.266 | H | C(CH₃)=NOCH₂CH=CHCH₃ | H | Cl |
| A.267 | H | C(CH₃)=NOCH₂CH≡CCH₃ | H | Cl |
| A.268 | H | CH₃ | H | CH₃ |
| A.269 | H | CF₃ | H | CH₃ |
| A.270 | H | OCH₃ | H | CH₃ |
| A.271 | H | OCF₃ | H | CH₃ |
| A.272 | H | cyclopropyl | H | CH₃ |
| A.273 | H | C₆H₅ | H | CH₃ |
| A.274 | H | O-C₆H₅ | H | CH₃ |
| A.275 | H | CH=NOCH₃ | H | CH₃ |
| A.276 | H | CH=NOCH(CH₃) | H | CH₃ |
| A.277 | H | CH=NOCH₂CH=CHCH₃ | H | CH₃ |
| A.278 | H | C(CH₃)=NOCH₃ | H | CH₃ |
| A.279 | H | C(CH₃)=NOCH₂CH=CHCH₃ | H | CH₃ |
| A.280 | H | C (CH₃) =NOCH₂CH≡CCH₃ | H | CH₃ |
| A.281 | H | CF₃ | H | CF₃ |
| A.282 | H | OCH₃ | H | CF₃ |
| A.283 | H | OCF₃ | H | CF₃ |
| A.284 | H | cyclopropyl | H | CF₃ |
| A.285 | H | C₆H₅ | H | CF₃ |
| A.286 | H | 2-F-C₆H₄ | H | CF₃ |
| A.287 | H | 2-CH₃-C₆H₄ | H | CF₃ |
| A.288 | H | 3-CF₃-C₆H₄ | H | CF₃ |
| A.289 | H | 4-OCH₃-C₆H₄ | H | CF₃ |
| A.290 | H | 3-OCF₃-C₆H₄ | H | CF₃ |
| A.291 | H | O-C₆H₅ | H | CF₃ |
| A.292 | H | O-[4-F-C₆H₄] | H | CF₃ |
| A.293 | H | O-[2-Cl-C₆H₄] | H | CF₃ |
| A.294 | H | O-[2-CH₃-C₆H₄] | H | CF₃ |
| A.295 | H | CH=NOCH₃ | H | CF₃ |
| A.296 | H | CH=NOCH(CH₃) | H | CF₃ |
| A.297 | H | CH=NOCH₂CH=CHCH₃ | H | CF₃ |
| A.298 | H | C(CH₃)=NOCH₃ | H | CF₃ |
| A.299 | H | C(CH₃)=NOCH₂CH=CHCH₃ | H | CF₃ |
| A.300 | H | C(CH₃)=NOCH₂CH≡CCH₃ | H | CF₃ |
| A.301 | H | OCH₃ | H | OCH₃ |
| A.302 | H | OCF₃ | H | OCH₃ |
| A.303 | H | cyclopropyl | H | OCH₃ |
| A.304 | H | C₆H₅ | H | OCH₃ |
| A.305 | H | O-C₆H₅ | H | OCH₃ |
| A.306 | H | CH=NOCH₃ | H | OCH₃ |
| A.307 | H | CH=NOCH(CH₃) | H | OCH₃ |
| A.308 | H | CH=NOCH₂CH=CHCH₃ | H | OCH₃ |
| A.309 | H | C(CH₃)=NOCH₃ | H | OCH₃ |
| A.310 | H | C(CH₃)=NOCH₂CH=CHCH₃ | H | OCH₃ |
| A.311 | H | C(CH₃)=NOCH₂CH≡CCH₃ | H | OCH₃ |
| A.312 | H | cyclopropyl | H | OCF₃ |
| A.313 | H | C₆H₅ | H | OCF₃ |
| A.314 | H | O-C₆H₅ | H | OCF₃ |
| A.315 | H | CH=NOCH₃ | H | OCF₃ |
| A.316 | H | CH=NOCH(CH₃) | H | OCF₃ |
| A.317 | H | CH=NOCH₂CH=CHCH₃ | H | OCF₃ |
| A.318 | H | C(CH₃)=NOCH₃ | H | OCF₃ |
| A.319 | H | C(CH₃)=NOCH₂CH=CHCH₃ | H | OCF₃ |
| A.320 | H | C(CH₃)=NOCH₂CH≡CCH₃ | H | OCF₃ |
| | | | | |
| A.321 | CH₃ | H | H | H |
| A.322 | Cl | H | H | H |
| A.323 | F | H | H | H |
| A.324 | CF₃ | H | H | H |
| A.325 | Cl | H | Cl | Cl |
| A.326 | CH₃ | CF₃ | H | H |
| A.327 | Cl | CF₃ | H | H |
| A.328 | F | CF₃ | H | H |
| A.329 | CH₃ | H | CF₃ | H |
| A.330 | CH₃ | H | Cl | H |
| A.331 | F | H | CF₃ | H |
| A.332 | F | H | F | H |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der Ascomyceten, Deuteromyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudocercosporella-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia inaequalis (Schorf) an Äpfeln.

Die Verbindungen I eignen sich außerdem zur Bekämpfung von Schadpilzen wie Paecilomyces variotii im Materialschutz (z.B. Holz, Papier, Dispersionen für den Ansprich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B.

Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben. Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäure- diamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-ylharnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(pchlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl) -acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.
1. Herstellung von 2,6-Dimethylphenyl-glyoxylsäure-methylester 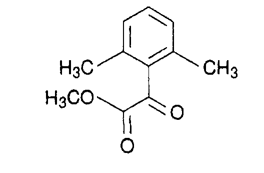
   Eine Grignard-Lösung aus 18,2 g (0,76 mol) Magnesiumspänen, 140 g (0,76 mol) Brom-meta-xylol und 500 ml Tetrahydrofuran wurde bei Raumtemperatur mit einer Mischung aus 109 g (0,8 mol) Zink-chlorid und 500 ml Tetrahydrofuran versetzt. Nach ca. 60 min. wurde der Reaktionsmischung bei 0°C bis -5°C tropfenweise eine Lösung aus 93 g (0,76 mol) Oxalsäuremethylester-chlorid und 250 ml Tetrahydrofuran zugegeben. Nach ca. 12 h bei Raumtemperatur wurde das Reaktionsgemisch unter Eiskühlung mit Wasser versetzt. Die so erhaltene Mischung wurde mehrmals mit Diethylether extrahiert. Die vereinigten organischen Phasen wurden mit Wasser und NaHCO₃-Lösung gewaschen, getrocknet und bei vermindertem Druck vom Lösungsmittel befreit. Der so erhaltene Rückstand wurde destillativ aufgereinigt. Man erhielt so 48 g der Titelverbindung als gelbliche, leicht bewegliche Flüssigkeit.
   - ¹H-NMR: (δ in ppm; CDCl₃): 2,2 (s, 6H); 3,8 (s, 3H); 7,0-7,3 (m, 3H).
2. Herstellung von 2,6-Dimethylphenyl-glyoxylsäure-methylester-O-methyloxim (E,Z-Gemisch) 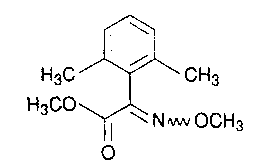
   Eine Mischung aus 19,2 g (0,1 mol) 2,6-Dimethylphenylglyoxylsäure-methylester und 100 ml Methanol wurde bei Raumtemperatur (ca. 25°C) tropfenweise mit 83,5 ml (0,15 mol) einer 15%-igen Lösung von O-Methylhydroxylamin-hydrochlorid in Methanol versetzt. Das so erhaltene Reaktionsgemisch wurde 16 h beim Siedepunkt des Lösungsmittels unter Rückfluß gerührt und anschließend mit weiteren 24 ml der 15%-igen Lösung von O-Methylhydroxylamin-hydrochlorid in Methanol versetzt. Nach weiteren 32 h beim Siedepunkt des Lösungsmittels wurde das Lösungsmittel bei vermindertem Druck entfernt. Der Rückstand wurde in Essigsäure-ethylester aufgenommen. Die organische Phase wurde mit Wasser gewaschen, getrocknet und bei vermindertem Druck vom Lösungsmittel befreit. Der so erhaltene Rückstand (20 g) wurde destillativ aufgereinigt. Man erhielt so 12 g der Titelverbindung, die ohne weitere Reinigung der Isomerisierung gemäß Beispiel 3 unterzogen werden können.
3. Herstellung von 2,6-Dimethylphenyl-glyoxylsäure-methylester-O-methyloxim (E-Isomer) 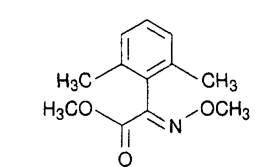
   In eine Mischung von 62 g (0,28 mol) des in Beispiel 2. erhaltenen Isomerengemisches (E/Z = 35/65) und 300 ml Methylenchlorid wurden bei 10°C 65 g (0,8 mol) HBr eingegast. Die Reaktionsmischung wurde für 60 h bei Raumtemperatur (ca. 25°C) gerührt, anschließend mit Wasser gewaschen und getrocknet. Die organische Lösung wurde bei vermindertem Druck vom Lösungsmittel befreit und der dabei verbleibende Rückstand wurde mit n-Heptan verrührt. Der dabei gebildete Feststoff wurde isoliert und getrocknet. Man erhielt so 32 g der Titelverbindung. (Isomerengehalt > 90%; GC, HPLC und NMR Analyse).
   - ¹H-NMR: (δ in ppm; CDCl₃): 2,1 (s, 6H); 3,8 (s, 3H); 4,0 (s, 3H); 7,0-7,3 (m, 3H).
4. Herstellung von 2-Brommethyl-6-methyl-phenyl-glyoxylsäuremethylester-O-methyloxim (E-Isomer) 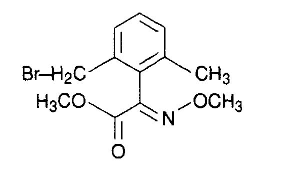
   Eine Mischung aus 43,3 g (0,196 mol) des in Beispiel 3 erhaltenen Produkts und 360 ml Tetrachlorkohlenstoff wurde mit 31,4 g (0,176 mol) N-Brom-succinimid und 0,9 g Azobisisobutyronitril versetzt. Das Reaktionsgemisch wurde für ca. 20 min. bei ca. 80°C gerührt, auf Raumtemperatur (ca. 25°C) gekühlt und von festen Rückständen befreit. Die organische Lösung wurde bei vermindertem Druck vom Lösungsmittel befreit und der dabei verbleibende Rückstand wurde mit Di-isopropylether gerührt. Der dabei gebildete Feststoff wurde isoliert, mit Di-isopropylether/Heptan (1:1) gewaschen und getrocknet. Man erhielt so 19,3 g der Titelverbindung; Fp.: 76-78°C. Das Filtrat wurde bei vermindertem Druck vom Lösungsmittel befreit und der dabei erhaltene Rückstand wurde chromatographisch [Kieselgel, Cyclohexan/tert.-Butyl-methylether (8:1)] gereinigt. Man erhielt so weitere 31,6 g der Titelverbindung.
   - ¹H-NMR: (δ in ppm; CDCl₃): 2,1 (s, 3H); 3,8 (s, 3H); 4,0 (s, 3H); 4,2 (s, 2H); 7,1-7,4 (m, 3H).
5. Herstellung von 2-{[(α-Methyl-3-trifluormethylbenzyl)imino] -oxymethyl}-6-methyl-phenylglyoxylsäure-methylester-O-methyloxim (E-Isomer) 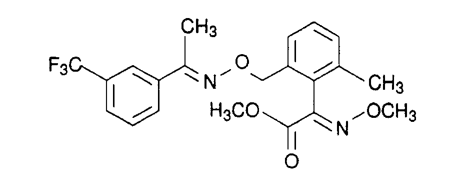
   Eine Mischung aus 1,1 g (5,4 mmol) 3-Trifluormethylacetophenon-oxim und 10 ml Dimthylformamid wurde mit 1,0 g (5,4 mmol) einer 30%-igen Natriummethylat-Lösung in Methanol versetzt. Nach ca. 30 min. bei Raumtemperatur (ca. 25°C) wurde das Reaktionsgemisch mit 1,6 g (5,9 mmol) der Verbindung aus Beispiel 4. versetzt. Nach weiteren 30 min. bei Raumtemperatur (ca. 25°C) wurde das so erhaltene Reaktionsgemisch auf Eiswasser gegossen. Die Mischung wurde mit tert.-Butylmethylether extrahiert. Die organischen Phasen wurden vereinigt, mit Wasser gewaschen, getrocknet und bei vermindertem Druck vom Lösungsmittel befreit. Der so erhaltene Rückstand wurde chromatographisch [Kieselgel; Cyclohexan/tert.-Butylmethylether 8:2) gereinigt. Man erhielt so 1,1 g der Titelverbindung als gelblichen Feststoff; Fp.: 63-66°C.
   - ¹H-NMR: (δ in ppm; CDCl₃): 2,1 (s, 3H); 2,2 (s, 3H); 3,8 (s, 3H); 4,1 (s, 3H); 5,1 (m, 2H); 7,2-8,0 (m, 7H).

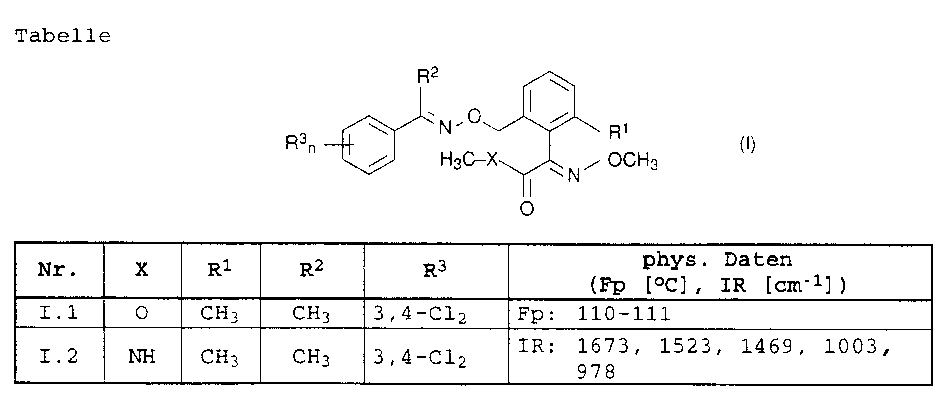

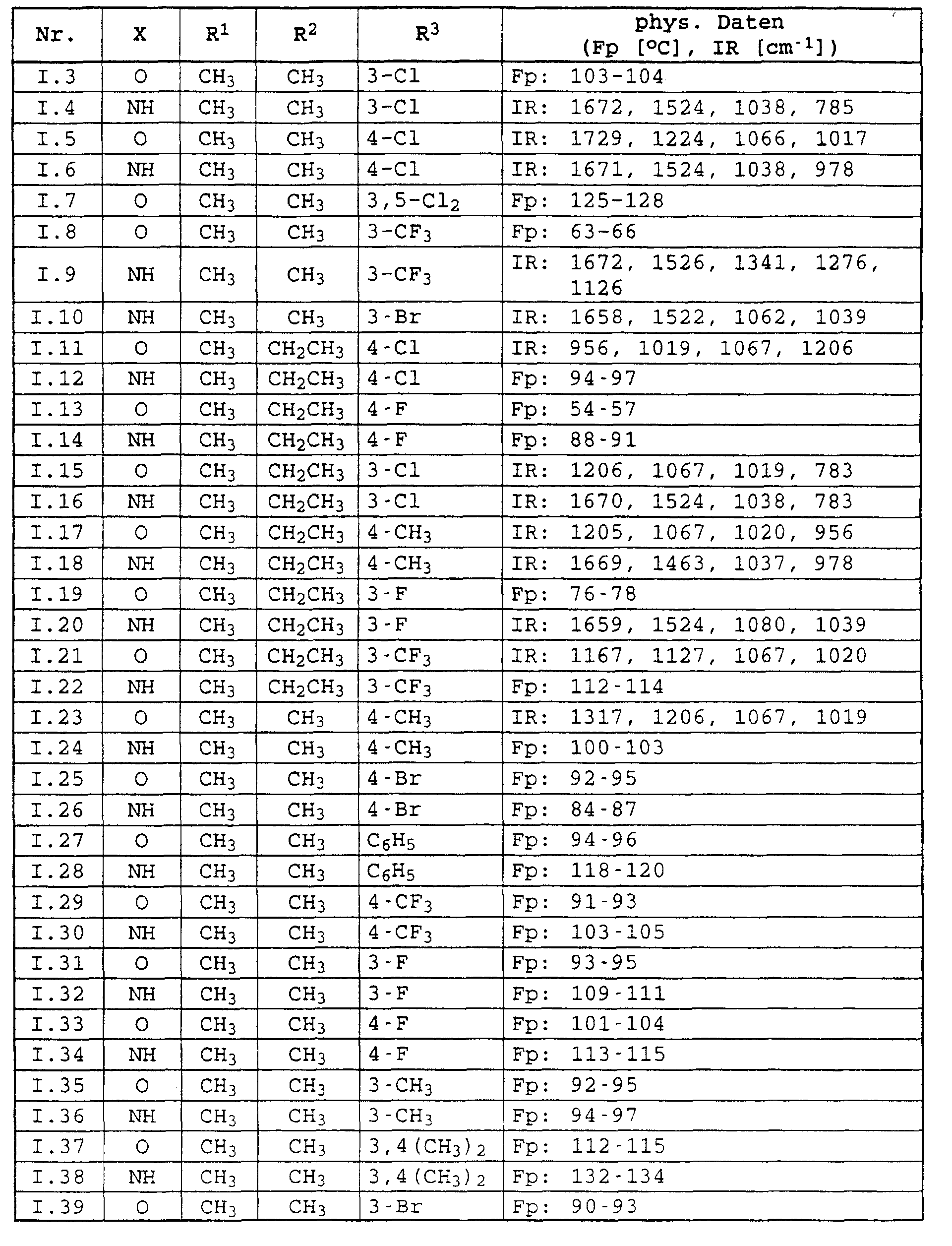

### Beispiele für die Wirkung gegen Schadpilze

Die verbesserte fungizide Wirkung der Verbindungen der allgemeinen Formel I ließ sich durch die folgenden Versuche zeigen. Als Vergleichsverbindungen des Standes der Technik dienten die bekannten Wirkstoffe (A):

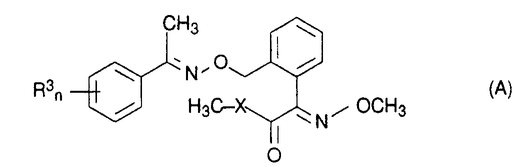

| **Nr.** | **R**^{**3**}_{**n**} | **X** |
|---|---|---|
| A.1 | 3-Cl | O |
| A.2 | 4-Cl | O |
| A.3 | 3,5-Cl₂ | O |
| A.4 | 3,4-Cl₂ | O |
| A.5 | 3-Cl | NH |
| A.6 | 4-Cl | NH |

Die Wirkstoffe wurden als 10%ige Emulsion in einem Gemisch aus 63 Gew.-% Cyclohexanon und 27 Gew.-% Emulgator aufbereitet und entsprechend der gewünschten Konzentration mit Wasser verdünnt.

### Anwendungsbeispiel 1:

### Wirksamkeit gegen Botrytis cinerea an Paprika

Paprikasämlinge der Sorte "Neusiedler Ideal Elite" wurden, nachdem sich 4 - 5 Blätter gut entwickelt hatten, mit einer wäßrigen Wirkstoffaufbereitung *(Aufwandmenge: 250 ppm)* bis zur Tropfnässe besprüht. Am nächsten Tag wurden die behandelten Pflanzen mit einer Sporensuspension von *Botrytis cinerea,* die 1.7 x 10⁶ Sporen/ml in einer 2%-igen wäßrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefall auf den Blättern visuell in % ermittelt werden.

| **Wirkstoff Nr.** | **Pilzbefall [%]** |
|---|---|
| I.3 | 5 |
| *A.1* | *60* |
| I.5 | 15 |
| *A.2* | *80* |
| I.7 | 15 |
| *A.3* | *60* |
| **unbehandelt** | **80** |

### Anwendungsbeispiel 2:

### kurative Wirksamkeit gegen Plasmopara viticola

Blätter von Topfreben der Sorte "Müller-Thurgau" wurden mit einer wäßrigen Zoosporenaufschwemmung von *Plasmopara viticola* inokuliert. Anschließend wurden die Reben für 48 Stunden in eine wasserdampfgesättigten Kammer bei 22-24° C gestellt. Dann wurden sie aus den Kammern herausgenommen und nach dem Abtrocknen mit wäßriger Wirkstoffaufbereitung *(Aufwandmenge: 63 ppm)* bis zur Tropfnäße besprüht. Nach dem Antrocknen des Spritzbelages wurden die Pflanzen für 5 Tage im Gewächshaus bei Temperaturen zwischen 20 und 30°C weiter kultiviert. Nach dieser Zeit wurden die Pflanzen zur Beschleunigung des Sporangienträgerausbruchs abermals für 16 Stunden in eine feuchte Klimakammer gestellt. Dann wurde das Ausmaß der Befallsentwicklung auf den Blattunterseiten visuell ermittelt.

| **Wirkstoff Nr.** | **Pilzbefall [%]** |
|---|---|
| I.1 | 25 |
| *A.4* | *40* |
| I.4 | 15 |
| *A.5* | *40* |
| I.6 | 25 |
| *A.6* | *80* |
| **unbehandelt** | **80** |

In einem entsprechenden Versuch zeigten die mit der Verbindung I.6 behandelten Pflanzen einen Befall von 5%.

### Beispiele für die Wirkung gegen tierische Schädlinge

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Phenylketiminooxybenzylverbindungen der allgemeinen Formel I 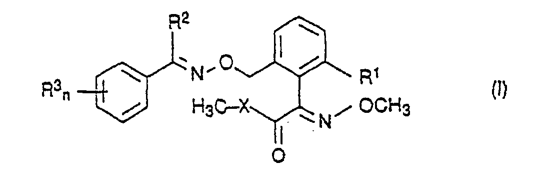 in der die Substituenten und der Index die folgende Bedeutung haben:
X O oder NH;
R¹ Fluor, Chlor, C₁-C₄-Alkyl, C₁-C₄-Halogenalkyl, C₁-C₄-Alkoxy und C₁-C₄-Halogenalkoxy;
R² Halogen, C₁-C₄-Alkyl, C₁-C₄-Halogenalkyl und C₁-C₄-Alkoxy;
R³ Halogen, C₁-C₄-Alkyl, C₁-C₄-Halogenalkyl, C₁-C₄-Alkoxy, C₁-C₄-Halogenalkoxy, C₃-C₆-Cycloalkyl, Phenyl oder Phenoxy, wobei die Phenylreste jeweils ihrerseits eine bis drei der folgenden Gruppen tragen können: Halogen, C₁-C₄-Alkyl, C₁-C₄-Halogenalkyl, C₁-C₄-Alkoxy und C₁-C₄-Halogenalkoxy;
CR^{a}=NOR^{b}, wobei
R^{a} für Wasserstoff oder C₁-C₄-Alkyl steht, und
R^{b} für C₁-C₄-Alkyl, C₃-C₄-Alkenyl oder C₃-C₄-Alkinyl steht;
n 1, 2 oder 3, wobei die Reste R³ verschieden sein können, wenn n 2 oder 3 bedeutet,
sowie ihre Salze.

2. Verfahren zur Herstellung der Verbindungen I gemäß Anspruch 1, **dadurch gekennzeichnet, daß** man ein N-Hydroxyketimin der Formel IIa 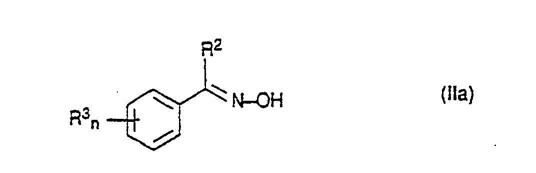 in einem inerten Lösungsmittel mit einer Benzylverbindung der Formel IIIa 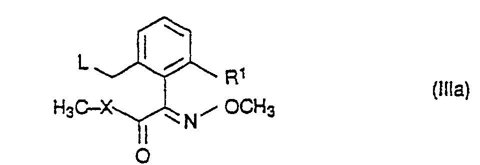 in der L für eine übliche Abgangsgruppe steht, umsetzt.

3. Verfahren zur Herstellung der Verbindungen I gemäß Anspruch 1, **dadurch gekennzeichnet, daß** man ein Phenylketon der Formel IIb, in der R² für Alkyl oder Halogenalkyl steht 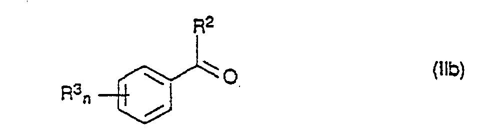 in einem inerten Lösungsmittel mit einem O-Benzylhydroxylamin der Formel IIIb 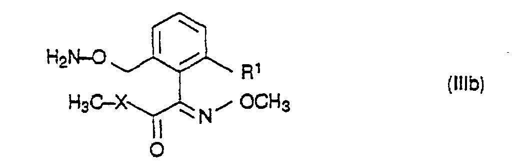 umsetzt.

4. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

5. Verfahren zur Bekämpfung von Schadpilzen, **dadurch gekennzeichnet, daß** man die Pilze oder die vor Pilzbefall zu schützenden Materialien, Pflanzen, den Boden oder die Saatgüter mit einer wirksamen Menge einer Verbindung der allgemeinen Formel I gemäß Anspruch 1 behandelt.

6. Verfahren zur Bekämpfung von tierischen Schädlingen, **dadurch gekennzeichnet, daß** man die Schädlinge oder die vor ihnen zu schützenden Räume, Materialien, Pflanzen, den Boden oder die Saatgüter mit einer wirksamen Menge einer Verbindung der allgemeinen Formel I gemäß Anspruch 1 behandelt.

7. Verwendung der Verbindungen I gemäß Anspruch 1 zur Herstellung eines zur Bekämpfung von tierischen Schädlingen oder Schadpilzen geeigneten Mittels.

8. Verwendung der Verbindungen I gemäß Anspruch 1 zur Bekämpfung von tierischen Schädlingen oder Schadpilzen.

## Claims

1. Phenylketiminooxybenzyl compounds of the general formula I 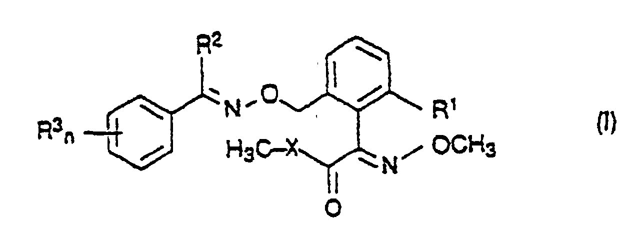 where:
X is O or NH;
R¹ is fluorine, chlorine, C₁-C₄-alkyl, C₁-C₄-haloalkyl, C₁-C₄-alkoxy and C₁-C₄-haloalkoxy;
R² is halogen, C₁-C₄-alkyl, C₁-C₄-haloalkyl and C₁-C₄-alkoxy;
R³ is halogen, C₁-C₄-alkyl, C₁-C₄-haloalkyl, C₁-C₄-alkoxy, C₁-C₄-haloalkoxy, C₃-C₆-cycloalkyl, phenyl or phenoxy, it being possible for each of the phenyl radicals in turn to carry one to three of the following groups: halogen, C₁-C₄-alkyl, C₁-C₄-haloalkyl, C₁-C₄-alkoxy and C₁-C₄-haloalkoxy;
CR^{a}=NOR^{b}, where
R^{a} is hydrogen or C₁-C₄-alkyl and
R^{b} is C₁-C₄-alkyl, C₃-C₄-alkenyl or C₃-C₄-alkynyl;
n is 1, 2 or 3, it being possible for the radicals R³ to be different if n is 2 or 3,
and salts thereof.

2. A process for preparing the compounds I as claimed in claim 1, which comprises reacting a N-hydroxyketimine of the formula IIa 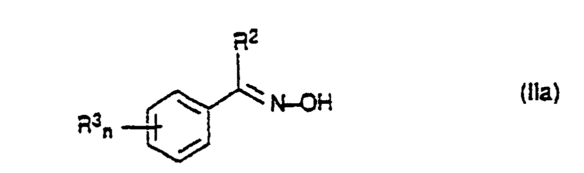 in an inert solvent with a benzyl compound of the formula IIIa 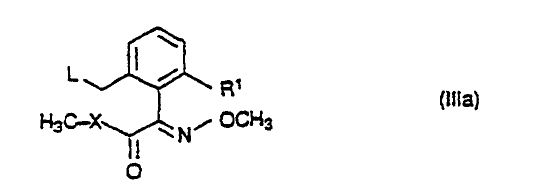 where L is a customary leaving group.

3. A process for preparing the compounds I as claimed in claim 1, which comprises reacting a phenyl ketone of the formula IIb, where R² is alkyl or haloalkyl, 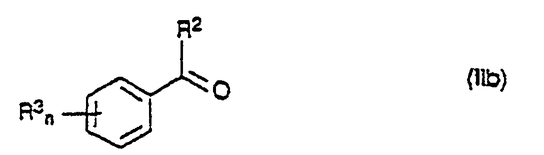 in an inert solvent with an O-benzylhydroxylamine of the formula IIIb.

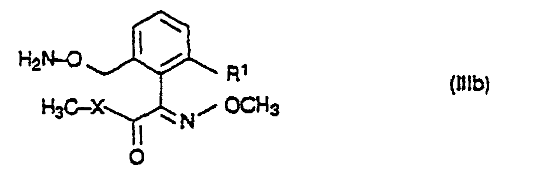

4. A composition suitable for controlling animal pests or harmful fungi comprising a solid or liquid carrier and a compound of the general formula I as claimed in claim 1.

5. A method for controlling harmful fungi, which comprises treating the fungi or the materials, plants, the soil or the seeds to be protected from being attacked by fungi with an effective amount of a compound of the general formula I as claimed in claim 1.

6. A method for controlling animal pests, which comprises treating the pests or the rooms, materials, plants, the soil or the seeds to be protected from them with an effective amount of a compound of the general formula I as claimed in claim 1.

7. The use of the compounds I as claimed in claim 1 for preparing a composition suitable for controlling animal pests or harmful fungi.

8. The use of the compounds I as claimed in claim 1 for controlling animal pests or harmful fungi.

## Revendications

1. Composés de type phénylcétimino-oxybenzyle de formule générale I 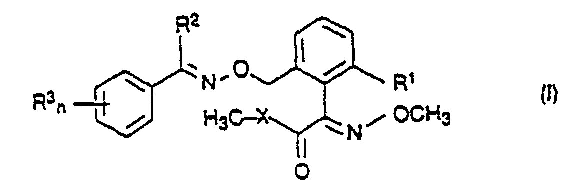 dans laquelle les substituants et l'indice ont les signifcations suivantes :
X représente O ou NH ;
R¹ représente un atome de fluor ou de chlore ou un groupe alkyle en C₁-C₄, halogénoalkyle en C₁-C₄, alcoxy en C₁-C₄ ou halogénoalcoxy en C₁-C₄ ;
R² représente un atome d'halogène ou un groupe alkyle en C₁-C₄, halogénoalkyle en C₁-C₄ ou alcoxy en C₁-C₄ ;
R³ représente un atome d'halogène ou un radical alkyle en C₁-C₄, halogénoalkyle en C₁-C₄, alcoxy en C₁-C₄, halogénoalcoxy en C₁-C₄, cycloalkyle en C₃-C₆, phényle ou phénoxy, les radicaux phényle pouvant pour leur part porter de un à trois des groupes suivants : halogéno, alkyle en C₁-C₄, halogénoalkyle en C₁-C₄, alcoxy en C₁-C₄ et halogénoalcoxy en C₁-C₄ ;
Cr^{a}=NOR^{b}, où
R^{a} représente un atome d'hydrogène ou un groupe alkyle en C₁-C₄, et
R^{b} représente un groupe alkyle en C₁-C₄, alcényle en C₃-C₄ ou alcynyle en C₃-C₄ ;
n représente 1, 2 ou 3, les radicaux R³ pouvant être différents lorsque n représente 2 ou 3,
ainsi que leurs sels.

2. Procédé pour la préparation des composés I selon la revendication 1, **caractérisé en ce qu'**on fait réagir une N-hydroxycétimine de formule IIa 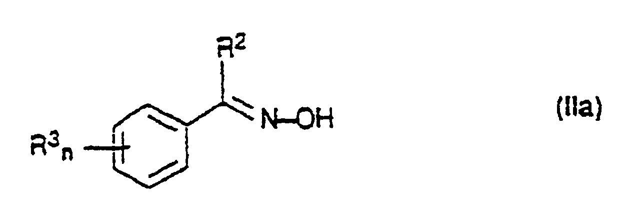 dans un solvant inerte, avec un composé benzylique de formule IIIa 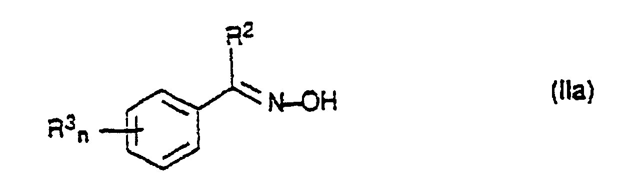 dans laquelle L représente un groupe partant usuel.

3. Procédé pour la préparation des composés I selon la revendication 1, **caractérisé en ce qu'**on fait réagir une phénylcétone de formule IIb, dans laquelle R² représente un groupe alkyle ou halogénoalkyle 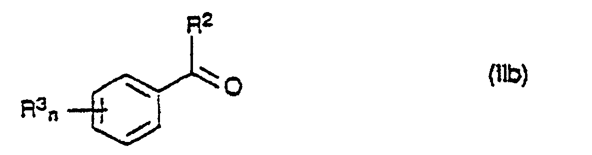 dans un solvant inerte, avec une O-benzylhydroxylamine de formule IIIb

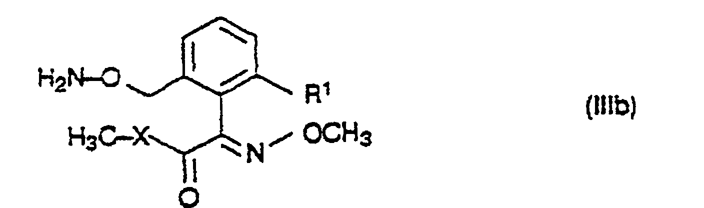

4. Composition appropriée à la lutte contre des champignons nuisibles ou des ravageurs animaux, contenant une substance de support solide ou liquide et un composé de formule générale I selon la revendication 1.

5. Procédé pour la lutte contre des champignons nuisibles, **caractérisé en ce qu'**on traite les champignons, ou les matériaux, les plantes, le sol ou les semences à protéger contre l'attaque de champignons, par une quantité efficace d'un composé de formule générale I selon la revendication 1.

6. Procédé pour la lutte contre des ravageurs animaux, **caractérisé en ce qu'**on traite les ravageurs ou les espaces, matériaux, plantes, le sol ou les semences à protéger contre ceux-ci, par une quantité efficace d'un composé de formule générale I selon la revendication 1.

7. Utilisation des composés I selon la revendication 1, pour la, préparation d'une composition appropriée à la lutte contre des champignons nuisibles ou des ravageurs animaux.

8. Utilisation des composés I selon la revendication 1, pour la lutte contre des champignons nuisibles ou des ravageurs animaux.
